# EUROPEAN PATENT SPECIFICATION

(11) **EP 4 538 272 B1**
(45) Date of publication and mention of the grant of the patent: **15.04.2026**
(21) Application number: 22945340.2
(22) Date of filing: 10.06.2022
(51) Int. Cl.: C07D 471/04, C07D 401/12, A61P 29/00, A61K 31/437, A61K 31/404, A61K 31/4439

(54) **CRYSTAL OF N-(BENZOYL)-PHENYLALANINE COMPOUND, AND PHARMACEUTICAL COMPOSITION THEREOF, AND PREPARATION METHOD THEREFOR AND USE THEREOF**
KRISTALL EINER N-(BENZOYL)-PHENYLALANINVERBINDUNG UND PHARMAZEUTISCHE ZUSAMMENSETZUNG DARAUS SOWIE HERSTELLUNGSVERFAHREN DAFÜR UND VERWENDUNG DAVON
CRISTAL DE COMPOSÉ DE N-(BENZOYL)-PHÉNYLALANINE, COMPOSITION PHARMACEUTIQUE DE CELUI-CI, PROCÉDÉ DE PRÉPARATION CORRESPONDANT ET UTILISATION ASSOCIÉE

(43) Date of publication of application: 16.04.2025
(73) Proprietor: Hangzhou Apeloa Medicine Research Institute Co., Ltd., Hangzhou, Zhejiang 310016 (CN); Apeloa Pharmaceutical Co., Ltd., Zhejiang 322118 (CN)
(72) Inventor: ZHAN, Weiqiang, Dongyang, Zhejiang 322118 (CN); FU, Lingyan, Dongyang, Zhejiang 322118 (CN); CHEN, Fanglei, Dongyang, Zhejiang 322118 (CN); TONG, Huan, Dongyang, Zhejiang 322118 (CN); WU, Jianbo, Dongyang, Zhejiang 322118 (CN); HE, Chun, Dongyang, Zhejiang 322118 (CN); XU, Xinliang, Dongyang, Zhejiang 322118 (CN); ZHU, Fangmeng, Dongyang, Zhejiang 322118 (CN)
(74) Representative: Perani & Partners S.p.A.
(86) International application number: PCT/CN2022/098176
(87) International publication number: WO 2023/236199

(56) References cited:
- EP-A1- 4 282 865
- WO-A1-2008/064823
- WO-A1-2022/156351
- CN-A- 114 222 730
- JP-A- 2016 037 467
- US-A1- 2003 220 318

## Description

### Field of the Invention

The present invention relates to the crystal pharmaceutical technology, specifically to crystals of N-(benzoyl)-phenylalanine compounds, pharmaceutical compositions comprising these crystal pharmaceuticals, as well as those preparation methods and purposes of the crystal pharmaceuticals. It particularly relates to two crystal forms of (S)-2-(2-chloro-6-fluorobenzoyl amido)-3-(4-(6',7' - difluoro-2'- oxospiro [cyclopropane-1,3'- indoline]-1'-yl) phenyl) propionic acid, corresponding pharmaceutical compositions and those preparation methods and purposes of the two crystal forms.

### Background of the Invention

IBDs (Inflammatory Bowel Diseases) are a series of chronic and inflammatory diseases that mainly affect the digestive tract. IBD includes ulcerative colitis (UC), Crohn's disease (CD) and indeterminate colitis. For example, when an IBD occurs and develops, immune cells can migrate towards the intestinal tract through the interaction between α4β7 integrin and its ligand mucosal addressin 1 (MAdCAM-1) and abnormally aggregate in the intestinal mucosal layer. The α4β7 integrin controls the transfer of lymphocytes towards the intestinal tissue and their retention in the intestinal tract through its interaction with MAdCAM-1.

It has been proposed that inhibiting the interaction between integrins and their ligands is an effective method for treating various autoimmune and inflammatory diseases, and blocking the interaction between α4β7 and MAdCAM-1 has shown its therapeutic effect on IBDs such as CD and UC.

PCT/CN2021/132456 indicates that a series of N - (benzoyl) - phenylalanine compounds with strong α4β7-MAdCAM-1 inhibitory activities can be used for prophylactizing and/or treating diseases (such as autoimmune and inflammatory diseases) related to α4β7 integrin, which includes the compound with a chemical name as (S)-2-(2-chloro-6-fluorobenzoyl amido)-3-(4-(6',7'-difluoro-2'-oxospiro [cyclopropane-1,3'-indoline]-1'-yl) phenyl) propionic acid. The preparation method of this compound is as follows: dissolve (S)-2-(2- chloro-6- fluorobenz amido)-3-(4-(6',7'-difluoro-2'-oxospiro [cyclopropane-1,3'- indoline]-1'-yl) phenyl) pmethyl propionate in tetrahydrofuran, and then add 0.5mol/L sodium hydroxide aqueous solution into the reaction system, let the reaction last for 2h at room temperature. Adjust the pH value of the reaction system to 1-2 with 2mol/L dilute hydrochloric acid, make an extraction three times (2mL each time) with dichloromethane, merge the organic layers, wash the final organic layer with water and saturated saline solution once, dry it with anhydrous sodium sulfate, filter and concentrate it under a reduced pressure. After the concentrate is purified by reverse phase HPLC (H₂O/CH₃CN system containing 0.1% formic acid), the compound shown by formula (I) can be obtained.

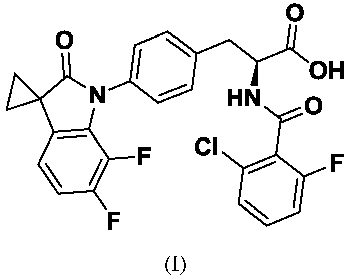

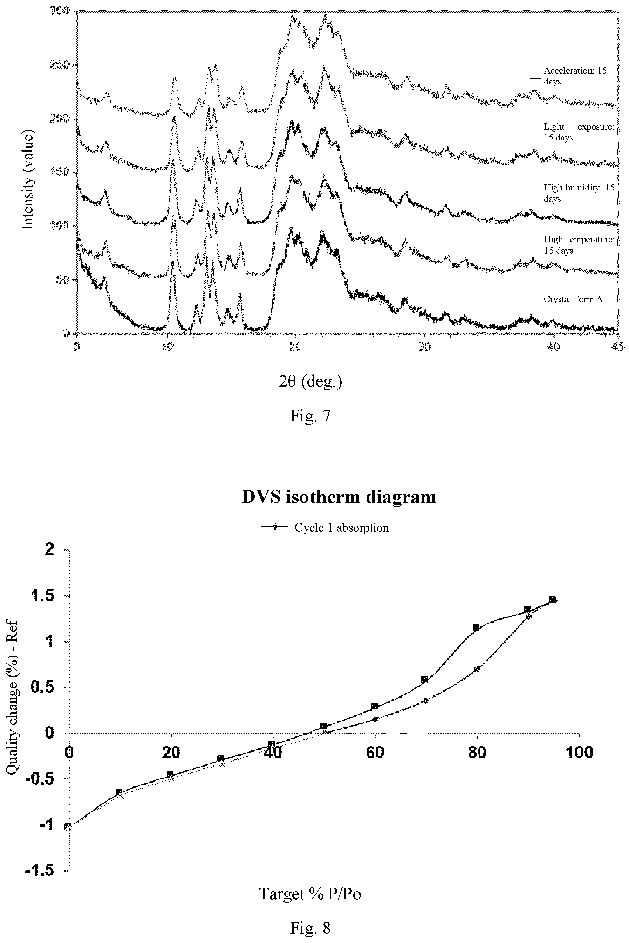

The product obtained according to the above method is amorphous (as shown in Fig. 7). Since amorphousness means relatively unstable solid forms in thermodynamics and is prone to transformation or degradation, a decrease in the chemical purity of compounds can be caused, affecting the final quality of pharmaceuticals.

### Description of the Invention

In response to the above deficiencies, for the present invention, it is unexpectedly found that Crystal Form A of the compound shown by Formula (I) and Crystal Form B of the compound shown by Formula (II) feature high stability, simple preparation process and are suitable for large-scale industrial production.

First, the present invention provides a compound shown by Formula (I), which exists in the form of Crystal Form A, and the crystallographic parameters of Crystal Form A are as follows:
Under Cu-Kα radiation, the X-ray powder diffraction (XRPD) pattern of Crystal Form A has characteristic peaks at 2θ values of 10.4+0.2°, 13.1±0.2°, 13.6±0.2°, 18.8+0.2°, 19.6+0.2°, 20.2±0.2°, 21.9±0.2° and 22.1±0.2°. Moreover, the XRPD pattern of Crystal Form A has three characteristic peaks with good peak shapes and resolutions at least at points with 20 values of 10.4±0.2°, 13.1+0.2° and 13.6±0.2°.

In some embodiments, the XRPD pattern of Crystal Form A also has at least one characteristic peak at points (e.g. 1, 2, 3, 4, 5, 6, 7 or 8 points) with 20 values of 5.2±0.2°, 12.3±0.2°, 14.7±0.2°, 15.7±0.2°, 24.7±0.2°, 26.5±0.2°, 28.5±0.2° and 33.1±0.2°.

In some embodiments, the XRPD pattern of Crystal Form A also has at least one characteristic peak at points (e.g. 1, 2 or 3 points) with 20 values of 31.6±0.2°, 38.3±0.2° and 40.0±0.2°.

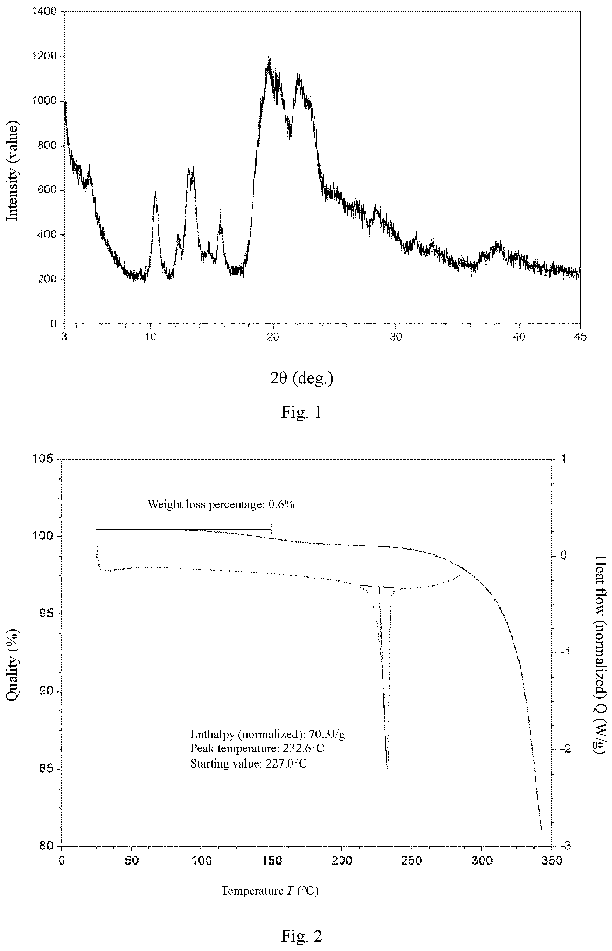

In some embodiments, the XRPD pattern of Crystal Form A is essentially as shown in Fig. 1.

In some embodiments, the differential scanning calorimetry (DSC) pattern of Crystal Form A has an endothermic peak at 236±3°C.

In some embodiments, the thermogravimetric analysis (TGA) pattern of Crystal Form A has a weight loss of approx. 0.2% at 25°C-120°C.

In some embodiments, the DSC / TGA patterns of Crystal Form A are essentially as shown in Fig. 2.

Second, the present invention provides a compound shown by Formula (II), which exists in the form of Crystal Form B, and the crystallographic parameters of Crystal Form B are as follows:
Under Cu-Kα radiation, the XRPD pattern of Crystal Form B has characteristic peaks at 2θ values of 8.5±0.2°, 11.0±0.2° and 17.9±0.2°.

In some embodiments, the XRPD pattern of Crystal Form B also has at least one characteristic peak at points (e.g. 1, 2, 3, 4, 5 or 6 points) with 2θ values of 14.3±0.2°, 15.5±0.2°, 19.3±0.2°, 20.1±0.2°, 24.4±0.2° and 25.5±0.2°.

In some embodiments, the XRPD pattern of Crystal Form B also has at least one characteristic peak at points (e.g. 1, 2, 3, 4, 5 or 6 points) with 20 values of 20.8±0.2°, 22.7±0.2°, 23.9±0.2°, 24.8±0.2°, 28.5±0.2° and 32.5±0.2°.

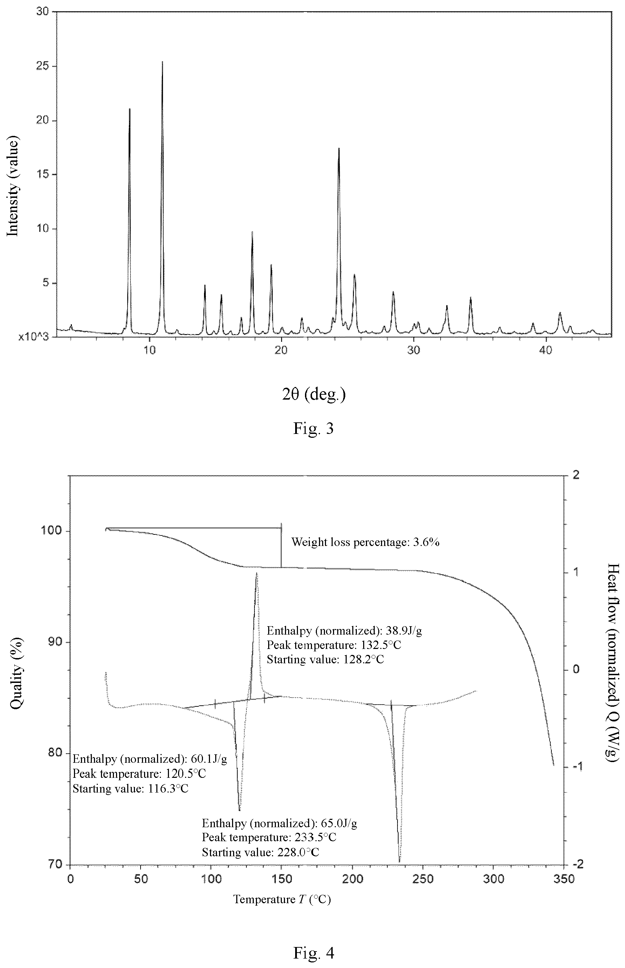

In some embodiments, the XRPD pattern of Crystal Form B is essentially as shown in Fig. 3.

In some embodiments, the DSC pattern of Crystal Form B has endothermic peaks at 121±3°C and 234±3°C, and an exothermic peak at 133±3°C.

In some embodiments, the TGA pattern of Crystal Form B shows a weight loss of approx. 3.6% at 50°C -150°C. According to the DSC analysis, Crystal Form B is a monohydrate crystal form.

In some embodiments, the DSC / TGA patterns of Crystal Form B are essentially as shown in Fig. 4.

Third, the present invention provides a method selected based on a suspension crystal transformation method for preparing the compound shown by formula (I) in the form of Crystal Form A.

In some embodiments, the steps of the above mentioned suspension crystal transformation method are: adding the compound shown by Formula (I) in an amorphous form to a binary mixed solvent to prepare a suspension, stirring it at a constant temperature and then performing solid-liquid separation (centrifugal separation preferred), drying the obtained solid (vacuum drying preferred), and obtaining the compound shown by Formula (I) in the form of Crystal Form A.

In some embodiments, the binary mixed solvent includes ethylene glycol monomethyl ether/methyl tert-butyl ether, ethylene glycol dimethyl ether/methyl tert butyl ether, 4-methyl-2-pentanone/water and acetone/water.

In some embodiments, the binary mixed solvent is ethylene glycol monomethyl ether/methyl tert butyl ether or acetone/water, with acetone/water being preferred.

In some embodiments, the volume ratio of the two solvents in the binary mixed solvent is 1:3-1:9, with 1:4-1:5 being preferred, and 1:5 being more preferred.

In some embodiments, the dosage ratio (solid-liquid ratio) of the compound shown by Formula (I) in an amorphous form to the binary mixed solvent is 30-80mg: 1ml, with 40-80mg:1ml being preferred, and 40mg:1ml being more preferred.

In some embodiments, the temperature for constant temperature stirring is from room temperature to 60°C, with 50-60°C being preferred, and 50°C being more preferred.

In some embodiments, the duration for constant temperature stirring is 4-120h, with 8-24h being preferred, and 8h being more preferred.

Fourth, the present invention provides a method selected based on a solvent evaporation method and an anti-solvent method for preparing the compound shown by Formula (II) in the form of Crystal Form B.

In some embodiments, the steps of the above mentioned solvent evaporation method are:
Adding the compound shown by Formula (I) in an amorphous form to an alcohol solvent for dissolving, and then let it stand for evaporation at room temperature to obtain the compound shown by formula (II) in the form of Crystal Form B.

In some embodiments, the alcohol solvent is at least methanol or ethanol, with ethanol being preferred.

In some embodiments, the dosage ratio (solid-liquid ratio) of the compound shown by formula (I) in an amorphous form to the alcohol solvent is 20-80mg:1ml, with **50mg:1ml** being preferred.

In some embodiments, the steps of the above mentioned anti-solvent method are:
Adding the compound shown by Formula (I) in an amorphous form to a good solvent for dissolving, and then adding the obtained solution to the anti-solvent, after stirring, performing solid-liquid separation (centrifugal separation preferred), drying the obtained solid (vacuum drying preferred), and obtaining the compound shown by Formula (II) in the form of Crystal Form B.

In some embodiments, the good solvent is at least methanol or ethanol, with ethanol being preferred.

In some embodiments, the anti-solvent is at least cyclohexane or n-hexane, with cyclohexane being preferred.

In some embodiments, the dosage ratio (solid-liquid ratio) of the compound shown by Formula (I) in an amorphous form to the good solvent is 40-70mg:1ml, with 55mg: 1ml being preferred.

In some embodiments, the dosage ratio (volume ratio) of anti-solvent to good solvent is 5-15:1, with 10:1 being preferred.

In some embodiments, the stirring operation includes stirring at room temperature and optional ice bath stirring, with the stirring duration of 0.5-3h at room temperature being preferred, and 1h being more preferred.

Fifth, the present invention provides a pharmaceutical composition comprising an effective prophylactic and/or therapeutic amount of a compound shown by Formula (I) in Crystal Form A and/or a compound of Formula (II) in Crystal Form B, as well as at least one pharmaceutically acceptable carrier.

Preferably, the pharmaceutically acceptable carrier is an inert, non-toxic carrier, which may include diluents, adhesives, disintegrants, flow aids, lubricants, coating agents and so on regarding starch, lactose, powdered cellulose, microcrystalline cellulose, arabic gum and so on used in pharmacy.

Sixth, the present invention provides the purposes of a compound shown by Formula (I) in Crystal Form A and/or a compound shown by Formula (II) in Crystal Form B in preparing pharmaceuticals for prophylactizing and/or treating diseases associated with α4β7 integrin.

The diseases associated with α4β7 integrin are autoimmune and inflammatory diseases; Preferably, the inflammatory diseases are IBDs such as UC and CD.

Crystal form A of the compound shown by Formula (I) and Crystal Form B of the compound shown by Formula (II) provided by the present invention have the following beneficial effects:
(1) Crystal Forms A and B of the present invention feature good stability
   The crystal form APIs (active pharmaceutical ingredients) provided by the present invention feature good physical and chemical stability under different storage conditions. The chemical purity and crystal forms of Crystal Forms A and B have no significant changes after being placed under a high temperature of 60°C, a high humidity of 92.5% RH, a light exposure of 4500lux and an acceleration condition of 40°C/75% RH for 15 days, respectively. This indicates that, for the present invention, Crystal Forms A and B as well as the pharmaceutical formulations containing Crystal Forms A and B can remain essentially unchanged during storage, ensuring the quality of the APIs and formulations.
(2) Crystal Forms A and B of the present invention feature low hygroscopicity
   Crystal Form A of the present invention has a weight gain of 0.09% under a relative humidity of 80%, indicating that no or almost no hygroscopicity can be found; Crystal Form B has a weight gain of 0.71% under a relative humidity of 80%, indicating that a slight hygroscopicity can be found. This means that Crystal forms A and B of the present invention are not prone to be affected by high humidity and deliquescence, improving the pharmaceutical stability, processing flowability and uniformity and enhancing the quality of pharmaceutical formulations.
(3) Crystal Forms A and B of the present invention feature good solubility
   Crystal Forms A and B of the present invention feature good solubility in FeSSIF (simulated intestinal fluid during feeding) and FaSSIF (simulated intestinal fluid during fasting). A good intestinal solubility is helpful for a good absorption of pharmaceuticals, improving their bioavailability and efficacy.
(4) The preparation process for Crystal Forms A and B of the present invention features good reproducibility, simple operation and is suitable for industrial production.

In summary, Crystal Forms A and B of the present invention have important application values in preparing pharmaceuticals for prophylactizing and/or treating diseases (e.g. autoimmune and inflammatory diseases) associated with α4β7 integrin.

### Description of the Drawings

Fig. 1 shows the XPRD pattern for Crystal Form A of the compound shown by Formula (I) prepared in Embodiment 1.
Fig. 2 shows the DSC / TGA patterns for Crystal Form A of the compound shown by Formula (I) prepared in Embodiment 1.
Fig. 3 shows the XPRD pattern for Crystal Form B of the compound shown by Formula (II) prepared in Embodiment 3.
Fig. 4 shows the DSC / TGA patterns for Crystal Form B of the compound shown by Formula (II) prepared in Embodiment 3.
Fig. 5 shows the XPRD pattern for the amorphous powder of the compound (I) prepared in Example 15 of PCT/CN2021/132456.
Fig. 6 shows the DVS pattern for Crystal Form A of the compound shown by Formula (I) prepared in Embodiment 6.
Fig. 7 shows the XPRD pattern for Crystal Form A of the compound shown by Formula (I) prepared in Embodiment 7 under high temperature, high humidity, light exposure and acceleration tests for 15 days.
Fig. 8 shows the DVS pattern for Crystal Form B of the compound shown by Formula (II) prepared in Embodiment 9.
Fig. 9 shows the XPRD pattern for Crystal Form B of the compound shown by Formula (II) prepared in Embodiment 10 under high temperature, high humidity, light exposure and acceleration tests for 15 days.

### Detailed Description of the Embodiments

The XRPD method adopted in the present invention involves: analyzing with a Bruker D8 Advance diffractometer, obtaining X-ray powder diffraction patterns under 40KV, 40Ma and Cu-Kα radiation and testing the sample should be tested at room temperature by placing it on a phosphide silicon wafer. The detailed testing conditions are: scanning within 3-45° with a step size of 0.02° and an exposure duration of 0.08s, collecting data through Diffrac. Measurement Center, and processing the data through Diffrac.Eva.

The DSC method adopted in the present invention involves: using a TA Discovery 2500 instrument with a thermal analysis controller to perform a DSC analysis, collecting and analyzing data through Trios, weighing approximately 1-2mg of sample accurately and placing it in a DSC Tzero sample tray with holes, analyzing the sample with a 10°C/min linear heating device under 25°C ~ 290°C, and purging the DSC furnace chamber during the operation with dry nitrogen at 50ml/min.

The TGA method adopted in the present invention involves: using a TA Discovery 55 instrument with a thermal analysis controller to perform a thermogravimetric analysis, collecting and analyzing data through Trios, placing approximately 2-5mg of sample in a balanced aluminum sample tray and automatically weighing it in a TGA heating furnace, analyzing the sample with a 10°C/min linear heating device under 25°C ~ 290°C, and purging the DSC chamber with dry nitrogen. The purging speeds at the sample and balance are 60mL/min and 40mL/min, respectively.

The solubility results in the present invention can be measured using an SHIMADZU LC 2030C 3D Plus high-performance liquid chromatograph; chromatographic column model: YMC Pack ODS-AQ C18, 4.6 ×250 mm, 5µm; measuring wavelength: 220nm; flow rate: 1.2ml/min; column temperature: 30°C; and mobile phase: 0.1% phosphoric acid solution - acetonitrile gradient elution.

The hygroscopicity results in the present invention can be measured using a DVS Intrinsic dynamic moisture and gas absorber from Surface Measurement Systems of the UK. Humidity variation: 50%-95%-0%-50%. The humidity change for each gradient within0% ~ 90% is 10%. Airflow: 200ml/min; temperature: 25°C; testing point: one testing point taken every 10% increase in humidity.

The following will provide a clear technical solution of the present invention in conjunction with the embodiments. Generally, those skilled in the art can understand that the following embodiments are only intended for further detailing the present invention rather than proposing a limitation. Unless otherwise limited, the pharmaceuticals, reagents, materials, instruments and so on used in the following embodiments can be obtained through conventional commercial means.

### Embodiment 1 Preparation of Crystal Form A

Weigh 40mg of the sample (the compound shown by Formula (I) in an amorphous form), add 0.1ml of ethylene glycol monomethyl ether and 0.4ml of methyl tert butyl ether to prepare a suspension, suspend and stir the solution at 50°C for 1 day, centrifuge the suspension, and dry the solid under vacuum at room temperature to obtain Crystal Form A. The XRPD pattern is shown in Fig. 1, and the DSC / TGA patterns are shown in Fig. 2.

**Table 1**

| No. | Diffraction angle 2θ | d value | Intensity % |
|---|---|---|---|
| 1 | 5.152° | 17.13880 | 18.6 |
| 2 | 10.447° | 8.46068 | 75.4 |
| 3 | 12.311° | 7.18391 | 28.3 |
| 4 | 13.082° | 6.76188 | 74.2 |
| 5 | 13.597° | 6.50719 | 69.1 |
| 6 | 14.719° | 6.01349 | 18.4 |
| 7 | 15.693° | 5.64239 | 36.6 |
| 8 | 18.808° | 4.71426 | 64.6 |
| 9 | 18.843° | 4.70576 | 62.5 |
| 10 | 19.574° | 4.53153 | 100.0 |
| 11 | 20.190° | 4.39473 | 88.4 |
| 12 | 21.858° | 4.06293 | 76.9 |
| 13 | 22.092° | 4.02047 | 86.4 |
| 14 | 22.109° | 4.01731 | 82.9 |
| 15 | 24.743° | 3.59529 | 21.7 |
| 16 | 26.537° | 3.35628 | 19.4 |
| 17 | 28.451° | 3.13468 | 18.6 |
| 18 | 31.641° | 2.82551 | 7.9 |
| 19 | 33.108° | 2.70355 | 11.9 |
| 20 | 38.296° | 2.34842 | 7.4 |
| 21 | 40.004° | 2.25201 | 6.7 |

### Embodiment 2 Preparation of Crystal Form A

Weigh and add 504mg of the sample (the compound shown by Formula (I) in an amorphous form) into 12.6ml of acetone/water (V/V=1:5) solvent, suspend and stir the solution at 50°C for 8h, centrifuge the obtained white suspension, dry the solid under vacuum at 50°C to obtain Crystal Form A. According to the testing result, its XRPD pattern is essentially the same as that shown in Fig. 1.

### Embodiment 3 Preparation of Crystal Form B

Add 20mg of the sample (the compound shown by Formula (I) in an amorphous form) into 0.4ml of ethanol, dissolve and let the solution evaporate at room temperature for 5 days to obtain Crystal Form B. Its XRPD pattern is shown in Fig. 3, and the DSC / TGA patterns are shown in Fig. 4.

According to the testing result, Crystal Form B can be transformed into Crystal Form A at a temperature above 150°C.

**Table 2**

| No. | Diffraction angle 20 | d value | Intensity % |
|---|---|---|---|
| 1 | 4.084 | 21.61671 | 1.3 |
| 2 | 8.142 | 10.85096 | 2.0 |
| 3 | 8.530 | 10.35778 | 52.0 |
| 4 | 11.029 | 8.01590 | 100.0 |
| 5 | 12.155 | 7.27595 | 0.8 |
| 6 | 14.269 | 6.20208 | 18.0 |
| 7 | 14.957 | 5.91853 | 2.2 |
| 8 | 15.493 | 5.71464 | 17.8 |
| 9 | 16.208 | 5.46414 | 0.5 |
| 10 | 17.023 | 5.20444 | 2.9 |
| 11 | 17.852 | 4.96469 | 30.3 |
| 12 | 18.647 | 4.75470 | 2.9 |
| 13 | 19.279 | 4.60019 | 19.7 |
| 14 | 19.669 | 4.50997 | 0.8 |
| 15 | 20.108 | 4.41232 | 11.3 |
| 16 | 20.822 | 4.26266 | 5.7 |
| 17 | 21.051 | 4.21681 | 0.9 |
| 18 | 21.628 | 4.10559 | 4.5 |
| 19 | 22.084 | 4.02189 | 2.5 |
| 20 | 22.268 | 3.98905 | 1.8 |
| 21 | 22.676 | 3.91821 | 5.4 |
| 22 | 22.841 | 3.89022 | 1.9 |
| 23 | 23.153 | 3.83845 | 1.9 |
| 24 | 23.937 | 3.71461 | 7.6 |
| 25 | 24.386 | 3.64715 | 22.9 |
| 26 | 24.845 | 3.58076 | 9.7 |
| 27 | 25.516 | 3.48812 | 14.1 |
| 28 | 26.426 | 3.37010 | 3.3 |
| 29 | 26.895 | 3.31230 | 1.7 |
| 30 | 27.809 | 3.20551 | 1.5 |
| 31 | 28.510 | 3.12830 | 5.0 |
| 32 | 29.411 | 3.03447 | 0.7 |
| 33 | 29.845 | 2.99134 | 1.5 |
| 34 | 30.083 | 2.96815 | 3.4 |
| 35 | 30.398 | 2.93817 | 4.8 |
| 36 | 31.196 | 2.86479 | 2.4 |
| 37 | 32.317 | 2.76796 | 2.6 |
| 38 | 32.533 | 2.75006 | 8.4 |
| 39 | 33.429 | 2.67838 | 0.5 |
| 40 | 33.674 | 2.65941 | 0.8 |
| 41 | 34.340 | 2.60935 | 4.6 |
| 42 | 35.598 | 2.51995 | 0.5 |
| 43 | 36.176 | 2.48100 | 0.7 |
| 44 | 36.550 | 2.45650 | 1.5 |
| 45 | 36.915 | 2.43302 | 0.4 |
| 46 | 37.669 | 2.38604 | 0.7 |
| 47 | 38.760 | 2.32134 | 0.6 |
| 48 | 39.047 | 2.30496 | 2.3 |
| 49 | 40.013 | 2.25150 | 0.9 |
| 50 | 41.122 | 2.19332 | 1.9 |
| 51 | 41.504 | 2.17402 | 0.4 |
| 52 | 41.872 | 2.15575 | 2.1 |
| 53 | 42.946 | 2.10428 | 0.5 |
| 54 | 43.621 | 2.07326 | 1.8 |

### Embodiment 4 Preparation of Crystal Form B

Add 20mg of the sample (the compound shown by Formula (I) in an amorphous form) into 0.35ml of ethanol. After dissolving, add the sample solution into 3ml of cyclohexane. Stir the solution at room temperature for 1h and then stir it in an ice bath, centrifuge and dry the solid under vacuum at room temperature to obtain Crystal Form B. According to the testing result, its XRPD pattern is essentially the same as that shown in Fig. 4.

### Embodiment 5 Solubility of Crystal Form A

Add 20mg of the sample (the compound shown by Formula (I) in the form of Crystal Form A) into a 10ml conical tube, and then add 4ml of water or biological medium (FeSSIF (pH 5.0) or FaSSIF (pH 6.5)), respectively. Shake the solution at 37°C for 24h in a constant temperature bath at 1000rpm, and take samples at 0.5h, 2h and 24h, respectively. Filter the samples through an aquo-system microporous filter membrane and discard the initial filtrate to obtain the test solution.

Perform an HPLC detection for the test solution (20µL), and calculate the sample concentration according to the standard curve method. The results are shown in the table below.

**Table 3. Solubility test results of Crystal Form A in different media**

| Medium | | FaSSIF | FeSSIF |
|---|---|---|---|
| Solubility (µg/ml) | 0.5h | 2130 | 460 |
| | 2h | 2230 | 480 |
| | 24h | 2220 | 490 |

Conclusion: As shown in Table 3, the solubility results of Crystal form A are relatively good in FeSSIF and FaSIF.

### Embodiment 6 Hygroscopicity of Crystal Form A

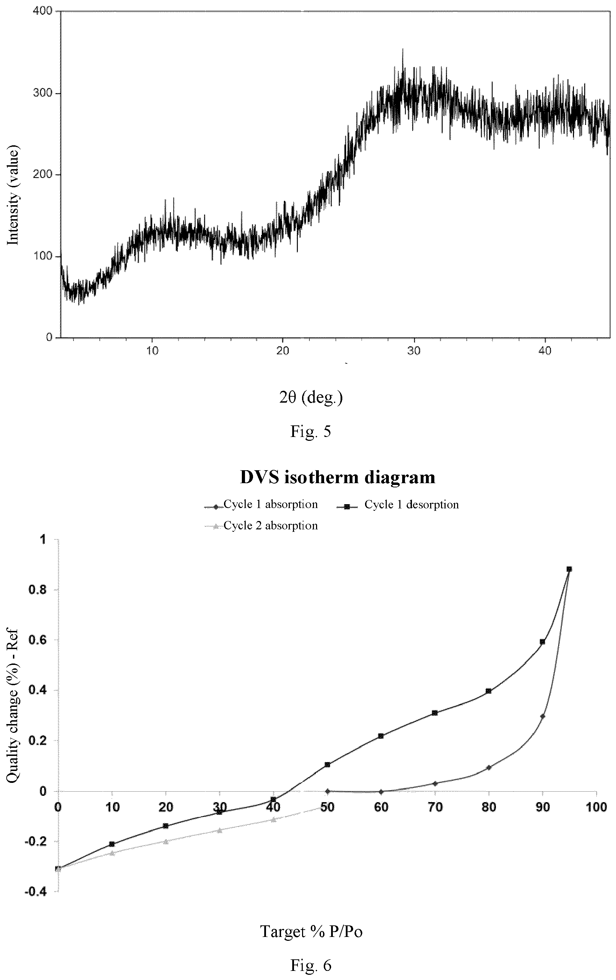

Take an appropriate amount of the test sample (the compound shown by Formula (I) in the form of Crystal Form A) to test its hygroscopicity with a dynamic moisture absorber. The test results are shown in Table 4, and the DVS pattern of the hygroscopicity test for Crystal Form A is shown in Fig. 6.

**Table 4. Hygroscopicity test results for Crystal Form A of the present invention**

| Test sample | Weight gain at 80% RH/% |
|---|---|
| Crystal Form A | 0.09% |

Conclusion: As shown in Table 4 and Fig. 6, the weight gain of Crystal Form A at 80% RH is 0.09%. According to the definition criteria for hygroscopic weight gain, no or almost no hygroscopicity can be found, indicating that Crystal Form A of the present invention is not prone to be affected by high humidity to cause deliquescence.

### Embodiment 7 Stability of Crystal Form A

Put an appropriate amount of the test sample (the compound shown by Formula (I) in the form of Crystal Form A) on a watch glass, spread it into a thin layer ≤ 5mm thick, and place it under a high temperature of 60°C, a high humidity of 92.5% RH, a light exposure of 4500lux and an acceleration condition of 40°C/75% RH for 15 days, respectively. Sample on the 7th and 15th day to observe the color change of the samples. Analyze the sample purity and crystal form through HPLC and XPRD detection, respectively. The test results are shown in Table 5, and the XRPD pattern is shown in Fig. 7.

**Table 5. Test results of Crystal Form A and amorphous stability**

| Test sample | | High temperature 60°C | High humidity 92.5%RH | Light exposure 4500lux | Acceleration 40°C/75%RH |
|---|---|---|---|---|---|
| Amorphous purity | 0d | 99.80% | 99.80% | 99.80% | 99.80% |
| | 15d | 91.89% | 93.06% | 94.91% | 89.70% |
| Crystal Form A purity | 0d | 99.5% | 99.5% | 99.5% | 99.55 |
| | 7d | 99.4% | 99.3% | 99.2% | 99.4% |
| | 15d | 99.4% | 99.3% | 99.2% | 99.4% |
| Form of Crystal Form A | 0d | / | / | / | / |
| | 7d | Unchanged | Unchanged | Unchanged | Unchanged |
| | 15d | Unchanged | Unchanged | Unchanged | Unchanged |

According to Table 5 and Fig. 7, after being kept for 15 days under a high temperature of 60°C, a high humidity of 92.5% RH, a light exposure of 4500lux, and an acceleration condition of 40°C/75% RH, no significant change in the purity and form of Crystal Form A is found. Compared with the amorphous form, Crystal Form A of the present invention has a higher stability and is suitable for medicinal use.

### Embodiment 8 Solubility of Crystal Form B

Add 20mg of the sample (the compound shown by Formula (II) in the form of Crystal Form B) into a 10ml conical tube, and then add 4ml of water or biological medium (FeSSIF (pH 5.0) or FaSSIF (pH 6.5)), respectively. Shake the solution at 37°C for 24h in a constant temperature bath at 1000rpm, and take samples at 0.5h, 2h and 24h, respectively. Filter the samples through an aquo-system microporous filter membrane and discard the initial filtrate to obtain the test solution.

Perform an HPLC detection for the test solution (20µL), and calculate the sample concentration according to the standard curve method. The results are shown in the table below.

**Table 6. Solubility test results of Crystal Form B in different media**

| Medium | | FaSSIF | FeSSIF |
|---|---|---|---|
| Solubility (µg/ml) | 0.5h | 3187 | 869 |
| | 2h | 2961 | 815 |
| | 24h | 2791 | 734 |

Conclusion: As shown in Table 6, the solubility results of Crystal form B are also relatively good in FeSSIF and FaSIF.

### Embodiment 9 Hygroscopicity of Crystal Form B

Take an appropriate amount of the test sample (the compound shown by Formula (II) in the form of Crystal Form B) to test its hygroscopicity with a dynamic moisture absorber. The test results are shown in Table 7, and the DVS pattern of the hygroscopicity test for Crystal Form B is shown in Fig. 8.

**Table 7. Hygroscopicity test results for Crystal Form B of the present invention**

| Test sample | Weight gain at 80% RH/% |
|---|---|
| Crystal Form B | 0.71% |

Conclusion: As shown in Table 7 and Fig. 8, the weight gain of Crystal Form B at 80% RH is 0.71%. According to the definition criteria for hygroscopic weight gain, a slight hygroscopicity can be found, indicating that Crystal Form B of the present invention is not prone to be affected by high humidity to cause deliquescence.

### Embodiment 10 Stability of Crystal Form B

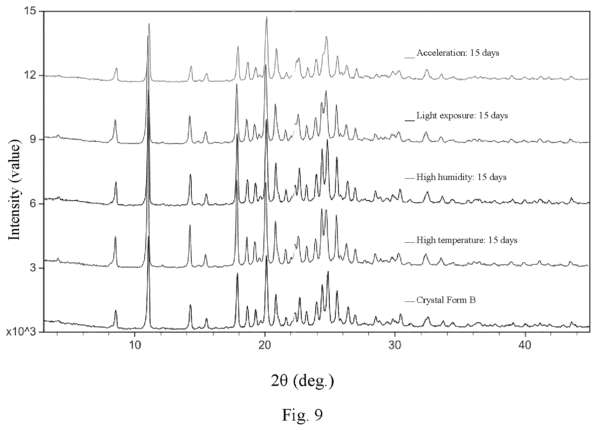

Put an appropriate amount of the test sample (the compound shown by Formula (II) in the form of Crystal Form B) on a watch glass, spread it into a thin layer ≤ 5mm thick, and place it under a high temperature of 60°C, a high humidity of 92.5% RH, a light exposure of 4500lux and an acceleration condition of 40°C/75% RH for 15 days, respectively. Sample on the 15th day to observe the color change of the sample. Analyze the sample purity and crystal form through HPLC and XPRD detection, respectively. The test results are shown in Table 8, and the XRPD pattern is shown in Fig. 9.

**Table 8. Test results of Crystal Form B stability**

| Test sample | | High temperature 60°C | High humidity 92.5%RH | Light exposure 4500lux | Acceleration 40°C/75%RH |
|---|---|---|---|---|---|
| Form of Crystal Form B | 0d | / | / | / | / |
| | 15d | Unchanged | Unchanged | Unchanged | Unchanged |
| Crystal Form B purity | 0d | 99.79% | 99.79% | 99.79% | 99.79% |
| | 15d | 99.80% | 99.80% | 99.80% | 99.80% |
| Amorphous purity | 0d | 99.80% | 99.80% | 99.80% | 99.80% |
| | 15d | 91.89% | 93.06% | 94.91% | 89.70% |

According to Table 8 and Fig. 9, after being kept for 15 days under a high temperature of 60°C, a high humidity of 92.5% RH, a light exposure of 4500lux, and an acceleration condition of 40°C/75% RH, no significant change in the purity and form of Crystal Form B is found. Compared with the amorphous form, Crystal Form B of the present invention has a higher stability and is suitable for medicinal use.

## Claims

1. A compound shown by Formula (I), 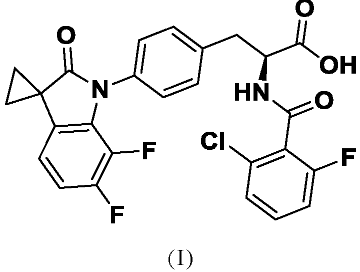 which exists in the form of crystal form A; under Cu-Kα radiation, an XRPD pattern of which shows characteristic peaks at 2θ values of 10.4+0.2°, 13.1±0.2°, 13.6±0.2°, 18.8±0.2°, 19.6±0.2°, 20.2±0.2°, 21.9±0.2° and 22.1±0.2°.

2. The compound shown by Formula (I) according to claim 1, wherein the XRPD pattern of which shows at least one of characteristic peaks at 2θ values of 5.2±0.2°, 12.3±0.2°, 14.7±0.2°, 15.7±0.2°, 24.7±0.2°, 26.5±0.2°, 28.5±0.2° and 33.14+0.2°;
preferably, the XRPD pattern of which shows at least one of characteristic peaks at 20 values of 31.6±0.2°, 38.3±0.2° and 40.0±0.2°;
more preferably, the XRPD pattern of which is essentially as shown in Fig. 1.

3. The compound shown by Formula (I) according to claim 1 or 2, wherein a DSC pattern of which shows an endothermic peak at 236±3°C;
preferably, the DSC pattern of which is essentially as shown in Fig. 2; and/or,
a TGA pattern of which shows a weight loss of approx. 0.2% at 25°C -120°C;
preferably, the TGA pattern of which is essentially as shown in Fig. 2.

4. A compound shown by Formula (II), 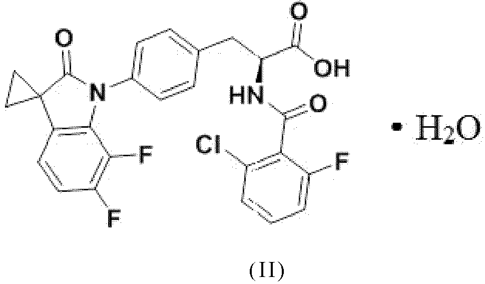 which exists in the form of crystal form B; under Cu-Kα radiation, an XRPD pattern of which shows characteristic peaks at 2θ values of 8.5±0.2°, 11.0±0.2° and 17.9±0.2°.

5. The compound shown by Formula (II) according to claim 4, wherein the XRPD pattern of which shows at least one of characteristic peaks at 20 values of 14.3±0.2°, 15.5±0.2°, 19.3+0.2°, 20.1±0.2°, 24.4±0.2° and 25.5±0.2°;
preferably, the XRPD pattern of which shows at least one of characteristic peaks at 20 values of 20.8±0.2°, 22.7±0.2°, 23.9±0.2°, 24.8±0.2°, 28.5±0.2° and 32.5±0.2°;
more preferably, the XRPD pattern of which is essentially as shown in Fig. 3.

6. The compound shown by Formula (II) according to claim 4 or 5, wherein a DSC pattern of which shows endothermic peaks at 121±3°C and 234±3°C, and an exothermic peak at 133±3°C;
preferably, the DSC pattern of which is essentially as shown in Fig. 4; and/or,
a TGA pattern of which shows a weight loss of approx. 3.6% at 50°C -150°C;
preferably, the TGA pattern of which is essentially as shown in Fig. 4.

7. A preparation method of the compound shown by Formula (I) according to any one of claims 1-3, wherein the method is suspension crystal transformation method.

8. A preparation method of the compound shown by Formula (II) according to any one of claims 4-6, wherein the method is selected from the group consisting of solvent evaporation method and anti-solvent method.

9. A pharmaceutical composition comprising a prophylactically and/or therapeutically effective amount of the compound shown by Formula (I) according to any one of claims 1-3 and/or the compound shown by Formula (II) according to any one of claims 4-6, as well as at least one pharmaceutically acceptable carrier.

10. The compound shown by Formula (I) according to any one of claims 1-3 and/or the compound shown by Formula (II) according to any one of claims 4-6 for use in preventing and/or treating diseases associated with α4β7 integrin;
preferably, the diseases associated with α4β7 integrin include autoimmune diseases and inflammatory diseases.

11. The compound shown by Formula (I) according to any one of claims 1-3 and/or the compound shown by Formula (II) according to any one of claims 4-6 for use as a medicament.

## Patentansprüche

1. Verbindung, die durch die Formel (I) dargestellt wird, 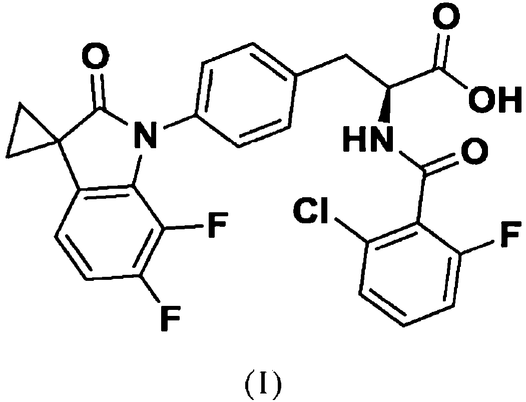 die in der Form der Kristallform A vorliegt; wobei unter Cu-Kα-Strahlung ein Röntgenpulverdiffraktogramm (XRPD-Diagramm) davon charakteristische Peaks bei 2θ-Werten von 10,4±0,2°, 13,1±0,2°, 13,6±0,2°, 18,8±0,2°, 19,6±0,2°, 20,2±0,2°, 21,9+40,2° und 22,1±0,2° aufweist.

2. Die durch die Formel (I) dargestellte Verbindung nach Anspruch 1, wobei das XRPD-Diagramm davon mindestens einen charakteristischen Peak bei 2θ-Werten von 5,2±0,2°, 12,3±0,2°, 14,7±0,2°, 15,7±0,2°, 24,7±0,2°, 26,5±0,2°, 28,5±0,2° und 33,1±0,2° aufweist;
vorzugsweise, wobei das XRPD-Diagramm davon mindestens einen charakteristischen Peak bei 2θ-Werten von 31,6±0,2°, 38,3±0,2° und 40,0±0,2° aufweist;
insbesondere, wobei das XRPD-Diagramm davon im Wesentlichen wie in Fig. 1 gezeigt ist.

3. Die durch die Formel (I) dargestellte Verbindung nach Anspruch 1 oder 2, wobei ein DSC-Diagramm (Differential-Scanning-Kalorimetrie) davon einen endothermen Peak bei 236±3°C aufweist;
vorzugsweise, wobei das DSC-Diagramm davon im Wesentlichen wie in Fig. 2 gezeigt ist; und/oder,
ein TGA-Diagramm (Thermogravimetrische Analyse) davon einen Gewichtsverlust von ca. 0,2 % bei 25°C - 120°C aufweist;
vorzugsweise, wobei das TGA-Diagramm davon im Wesentlichen wie in Fig. 2 gezeigt ist.

4. Verbindung, die durch die Formel (II) dargestellt wird, 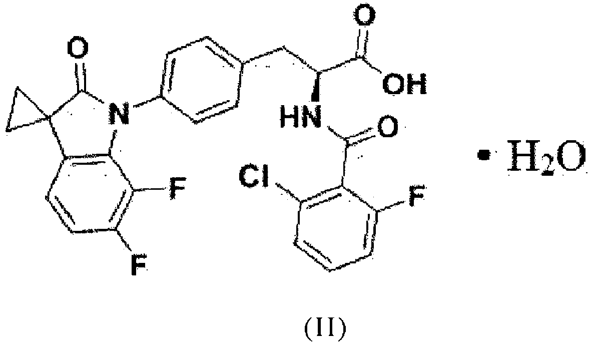 die in der Form der Kristallform B vorliegt; wobei unter Cu-Kα-Strahlung ein Röntgenpulverdiffraktogramm (XRPD-Diagramm) davon charakteristische Peaks bei 2θ-Werten von 8,5±0,2°, 11,0+0,2° und 17,9±0,2° aufweist.

5. Die durch die Formel (II) dargestellte Verbindung nach Anspruch 4, wobei das XRPD-Diagramm davon mindestens einen charakteristischen Peak bei 2θ-Werten von 14,3±0,2°, 15,5±0,2°, 19,3±0,2°, 20,1±0,2°, 24,4±0,2° und 25,5±0,2° aufweist;
vorzugsweise, wobei das XRPD-Diagramm davon mindestens einen charakteristischen Peak bei 20-Werten von 20,8±0,2°, 22,7±0,2°, 23,9±0,2°, 24,8±0,2°, 28,5±0,2° und 32,5±0,2° aufweist;
insbesondere, wobei das XRPD-Diagramm davon im Wesentlichen wie in Fig. 3 gezeigt ist.

6. Die durch die Formel (II) dargestellte Verbindung nach Anspruch 4 oder 5, wobei ein DSC-Diagramm davon endotherme Peaks bei 121±3°C und 234±3°C und einen exothermen Peak bei 133±3°C aufweist;
vorzugsweise, wobei das DSC-Diagramm davon im Wesentlichen wie in Fig. 4 gezeigt ist; und/oder,
ein TGA-Diagramm davon einen Gewichtsverlust von ca. 3,6 % bei 50°C - 150°C aufweist;
vorzugsweise, wobei das TGA-Diagramm davon im Wesentlichen wie in Fig. 4 gezeigt ist.

7. Herstellungsverfahren für die durch die Formel (I) dargestellte Verbindung nach einem der Ansprüche 1-3, wobei das Verfahren ein Suspensionskristalltransformationsverfahren ist.

8. Herstellungsverfahren für die durch die Formel (II) dargestellte Verbindung nach einem der Ansprüche 4-6, wobei das Verfahren aus der Gruppe ausgewählt ist, die aus einem Lösungsmittelverdampfungsverfahren und einem Antilösungsmittelverfahren besteht.

9. Pharmazeutische Zusammensetzung, umfassend eine prophylaktisch und/oder therapeutisch wirksame Menge der durch die Formel (I) dargestellten Verbindung nach einem der Ansprüche 1-3 und/oder der durch die Formel (II) dargestellten Verbindung nach einem der Ansprüche 4-6, sowie mindestens einen pharmazeutisch annehmbaren Träger.

10. Die durch die Formel (I) dargestellte Verbindung nach einem der Ansprüche 1-3 und/oder die durch die Formel (II) dargestellte Verbindung nach einem der Ansprüche 4-6 zur Verwendung bei der Vorbeugung und/oder Behandlung von mit α4β7-Integrin assoziierten Krankheiten;
vorzugsweise, wobei die mit α4β7-Integrin assoziierten Krankheiten Autoimmunerkrankungen und entzündliche Erkrankungen umfassen.

11. Die durch die Formel (I) dargestellte Verbindung nach einem der Ansprüche 1-3 und/oder die durch die Formel (II) dargestellte Verbindung nach einem der Ansprüche 4-6 zur Verwendung als Medikament.

## Revendications

1. Composé représenté par la Formule (I), 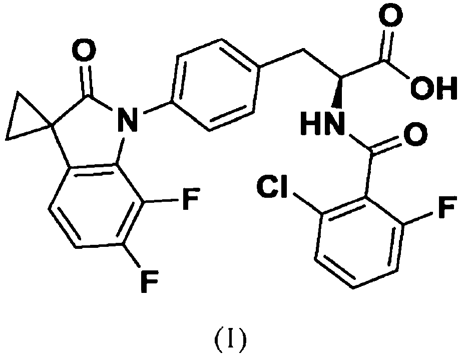 qui existe sous la forme de forme cristalline A ; sous un rayonnement Cu-Kα, un diagramme de diffraction des rayons X sur poudre (DRXP) de celui-ci présentant des pics caractéristiques à des valeurs 2θ de 10,4±0,2°, 13,1±0,2°, 13,6±0,2°, 18,8±0,2°, 19,6±0,2°, 20,2±0,2°, 21,9±0,2° et 22,1±0,2°.

2. Composé représenté par la Formule (I) selon la revendication 1, dans lequel le diagramme DRXP de celui-ci présente au moins un pic caractéristique aux valeurs 20 de 5,2±0,2°, 12,3±0,2°, 14,7±0,2°, 15,7±0,2°, 24,7±0,2°, 26,5±0,2°, 28,5±0,2° et 33,1±0,2°;
de préférence, le diagramme DRXP de celui-ci présentant au moins un pic caractéristique aux valeurs 2θ de 31,6±0,2°, 38,3±0,2° et 40,0±0,2°;
plus préférablement, le diagramme DRXP de celui-ci étant essentiellement tel que représenté sur la Fig. 1.

3. Composé représenté par la Formule (I) selon la revendication 1 ou 2, dans lequel un diagramme de calorimétrie différentielle à balayage (DSC) de celui-ci présente un pic endothermique à 236±3°C;
de préférence, le diagramme de DSC de celui-ci étant essentiellement tel que représenté sur la Fig. 2; et/ou,
un diagramme d'analyse thermogravimétrique (ATG) de celui-ci présentant une perte de poids d'environ 0,2 % à 25°C - 120°C;
de préférence, le diagramme d'ATG de celui-ci étant essentiellement tel que représenté sur la Fig. 2.

4. Composé représenté par la Formule (II), 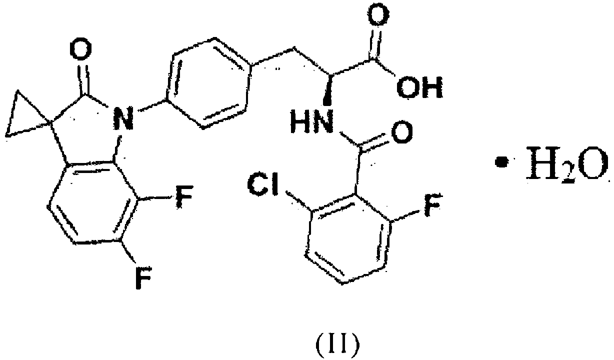 qui existe sous la forme de forme cristalline B; sous un rayonnement Cu-Kα, un diagramme de diffraction des rayons X sur poudre (DRXP) de celui-ci présentant des pics caractéristiques à des valeurs 2θ de 8,5±0,2°, 11,0±0,2° et 17,9±0,2°.

5. Composé représenté par la Formule (II) selon la revendication 4, dans lequel le diagramme DRXP de celui-ci présente au moins un pic caractéristique aux valeurs 2θ de 14,3±0,2°, 15,5±0,2°, 19,3±0,2°, 20,1±0,2°, 24,4±0,2° et 25,5±0,2°;
de préférence, le diagramme DRXP de celui-ci présentant au moins un pic caractéristique aux valeurs 2θ de 20,8±0,2°, 22,7±0,2°, 23,9±0,2°, 24,8±0,2°, 28,5±0,2° et 32,5±0,2°;
plus préférablement, le diagramme DRXP de celui-ci étant essentiellement tel que représenté sur la Fig. 3.

6. Composé représenté par la Formule (II) selon la revendication 4 ou 5, dans lequel un diagramme de DSC de celui-ci présente des pics endothermiques à 121±3°C et 234±3°C, et un pic exothermique à 133±3°C;
de préférence, le diagramme de DSC de celui-ci étant essentiellement tel que représenté sur la Fig. 4; et/ou,
un diagramme d'ATG de celui-ci présentant une perte de poids d'environ 3,6 % à 50°C - 150°C;
de préférence, le diagramme d'ATG de celui-ci étant essentiellement tel que représenté sur la Fig. 4.

7. Procédé de préparation du composé représenté par la Formule (I) selon l'une quelconque des revendications 1 à 3, dans lequel le procédé est un procédé de transformation cristalline en suspension.

8. Procédé de préparation du composé représenté par la Formule (II) selon l'une quelconque des revendications 4 à 6, dans lequel le procédé est choisi dans le groupe constitué par un procédé d'évaporation de solvant et un procédé anti-solvant.

9. Composition pharmaceutique comprenant une quantité prophylactiquement et/ou thérapeutiquement efficace du composé représenté par la Formule (I) selon l'une quelconque des revendications 1 à 3 et/ou du composé représenté par la Formule (II) selon l'une quelconque des revendications 4 à 6, ainsi qu'au moins un support pharmaceutiquement acceptable.

10. Composé représenté par la Formule (I) selon l'une quelconque des revendications 1 à 3 et/ou le composé représenté par la Formule (II) selon l'une quelconque des revendications 4 à 6 pour une utilisation dans la prévention et/ou le traitement de maladies associées à l'intégrine α4β7;
de préférence, les maladies associées à l'intégrine α4β7 incluent des maladies auto-immunes et des maladies inflammatoires.

11. Composé représenté par la Formule (I) selon l'une quelconque des revendications 1 à 3 et/ou le composé représenté par la Formule (II) selon l'une quelconque des revendications 4 à 6 pour une utilisation en tant que médicament.
